# EUROPEAN PATENT APPLICATION

(11) **EP 1 982 677 A2**
(43) Date of publication of application: **22.10.2008**
(21) Application number: 08153851.4
(22) Date of filing: 31.03.2008
(51) Int. Cl.: A61F 2/84

(54) **Delivery system for stent-graft**

(30) Priority: 19.04.2007 US 737385
(71) Applicant: Medtronic Vascular, Inc., Santa Rosa CA 95403 (US)
(72) Inventor: Murray, Robert, Santa Rosa, CA 95409 (US)
(74) Representative: Zimmermann & Partner

(57) **Abstract**

A delivery system for selectively holding and deploying each end of an endoprosthesis (1902) includes a spindle (120G) having a spindle body (122G). The spindle further includes proximal spindle pins (124G) and distal spindle pins (2030) extending radially outward from the spindle body. The delivery system further comprises a sleeve (112G) and a middle member sleeve (2022). The endoprosthesis (1902) includes proximal spindle pin catches (1202) and distal spindle pin catches (2012) at proximal and distal ends of the endoprosthesis. The distal spindle pins (2030) extend into the proximal spindle pin catches (1202) and the sleeve (112G) radially constrains the proximal end of the endoprosthesis. The proximal spindle pins (124G) extend into the distal spindle pin catches (2012) and the middle member sleeve (2022) radially constrains the distal end of the endoprosthesis. Spindle pins may be omitted at one or both ends of the endoprosthesis, in some configurations.

## Description

### BACKGROUND OF THE INVENTION

### Field of the Invention

This invention relates generally to medical devices and procedures, and more particularly to a method and system of deploying a stent-graft in a vascular system and to the associated stent-graft.

### Description of the Related Art

Prostheses for implantation in blood vessels or other similar organs of the living body are, in general, well known in the medical art. For example, prosthetic vascular grafts formed of biocompatible materials (e.g., Dacron or expanded, porous polytetrafluoroethylene (PTFE) tubing) have been employed to replace or bypass damaged or occluded natural blood vessels.

A graft material supported by a framework is known as a stent-graft or endoluminal graft. In general, the use of stent-grafts for treatment or isolation of vascular aneurysms and vessel walls which have been thinned or thickened by disease (endoluminal repair or exclusion) is well known.

Many stent-grafts are "self-expanding", i.e., inserted into the vascular system in a compressed or contracted state, and permitted to expand upon removal of a restraint. Self-expanding stent-grafts typically employ a wire or tube configured (e.g., bent or cut) to provide an outward radial force and employ a suitable elastic material such as stainless steel or Nitinol (nickel-titanium). Nitinol may additionally employ shape memory properties.

The self-expanding stent-graft is typically configured in a tubular shape of a slightly greater diameter than the diameter of the blood vessel in which the stent-graft is intended to be used. In general, rather than inserting in a traumatic and invasive manner, stents and stent-grafts are typically deployed through a less invasive intraluminal delivery, i.e., cutting through the skin to access a lumen or vasculature or percutaneously via successive dilatation, at a convenient (and less traumatic) entry point, and routing the stent-graft through the lumen to the site where the prosthesis is to be deployed.

Intraluminal deployment in one example is effected using a delivery catheter with coaxial inner tube, sometimes called the plunger, and outer tube, sometimes called the sheath, arranged for relative axial movement. The stent-graft is compressed and disposed within the distal end of the sheath in front of the inner tube.

The catheter is then maneuvered, typically routed though a lumen (e.g., vessel), until the end of the catheter (and the stent-graft) is positioned in the vicinity of the intended treatment site. The inner tube is then held stationary while the sheath of the delivery catheter is withdrawn. The inner tube prevents the stent-graft from moving back as the sheath is withdrawn.

As the sheath is withdrawn, the stent-graft is gradually exposed from a proximal end to a distal end of the stent-graft, the exposed portion of the stent-graft radially expands so that at least a portion of the expanded portion is in substantially conforming surface contact with a portion of the interior of the lumen, e.g., blood vessel wall.

The proximal end of the stent-graft is the end closest to the heart whereas the distal end is the end furthest away from the heart during deployment. In contrast and of note, the distal end of the catheter is usually identified to the end that is farthest from the operator (handle) while the proximal end of the catheter is the end nearest the operator (handle). For purposes of clarity of discussion, as used herein, the distal end of the catheter is the end that is farthest from the operator (the end furthest from the handle) while the distal end of the stent-graft is the end nearest the operator (the end nearest the handle), i.e., the distal end of the catheter and the proximal end of the stent-graft are the ends furthest from the handle while the proximal end of the catheter and the distal end of the stent-graft are the ends nearest the handle. However, those of skill in the art will understand that depending upon the access location, the stent-graft and delivery system description may be consistent or opposite in actual usage.

Many self expanding stent-graft deployment systems are configured to have the proximal end of the stent-graft deploy as the sheath is pulled back. The proximal end of the stent-graft is typically designed to fixate and seal the stent-graft to the wall of the vessel during deployment. Such a configuration leaves little room for error in placement since re-positioning the stent-graft after initial deployment, except for a minimal pull down retraction, is usually difficult if possible at all. Deploying the proximal end of the stent-graft first makes accurate pre-deployment positioning of the stent-graft critical.

Attempts to overcome this problem generally fail to provide adequate control in manipulating the stent-graft positioning in both the initial deployment of the stent-graft and the re-deployment of the stent-graft (once the stent-graft has been partially deployed).

Another problem encountered with existing systems, particularly with systems that have a distal end of a stent-graft fixed during deployment (or during the uncovering of the sheath) is the contact force between the retracting sheath and the stent graft contained therein make it necessary to use more retraction force to cause the stent-graft to axially compress or bunch up as the sheath is retracted. This bunching increases the density of the stent-graft within the sheath and can further increase the frictional drag experienced during deployment.

### SUMMARY OF THE INVENTION

In view of the above, a delivery system for an endoprosthesis provided which includes a spindle having a spindle body and spindle pins extending radially outward from the spindle body. The delivery system further includes a tapered tip having a sleeve, the spindle pins extending from the spindle body toward the sleeve. The endoprosthesis includes a proximal anchor stent ring having spindle pin catches and anchor pins. The spindle pins of the spindle extend into the spindle pin catches and the sleeve radially constrains the anchor pins.

A method of deploying the endoprosthesis includes radially constraining the proximal anchor stent ring of the endoprosthesis in an annular space between the sleeve of the tapered tip and the spindle. The method further includes radially constraining a graft material of the endoprosthesis in a primary sheath, the graft material being attached to a distal end of the proximal anchor stent ring. By radially constraining the graft material of the endoprosthesis by the primary sheath and radially constraining the proximal anchor stent ring by the sleeve, sequential and independent deployment of the graft material and the proximal anchor stent ring is facilitated.

The primary sheath is retracted to deploy a portion of the endoprosthesis. As the endoprosthesis is only partially deployed and the proximal anchor stent ring is radially constrained and un-deployed, the endoprosthesis can be repositioned in the event that the initial positioning of the endoprosthesis is less than desirable.

Further, as the proximal end of the endoprosthesis is secured and, in one example, the distal end is free to move within the primary sheath, bunching of the endoprosthesis during retraction of the primary sheath is avoided. By avoiding bunching, of the endoprosthesis on the primary sheath during retraction is minimized thus facilitating smooth and easy retraction of the primary sheath.

Once the endoprosthesis is properly positioned, the tapered tip is advanced to deploy the proximal anchor stent ring thus anchoring the endoprosthesis in position within the vessel. The anchor pins of the proximal anchor stent ring protrude radially outward and penetrate into the vessel wall, e.g., into healthy strong tissue.

In accordance with one example, the proximal anchor stent ring of the endoprosthesis includes proximal apexes, distal apexes, and struts extending between the proximal apexes and the distal apexes. The struts, the proximal apexes, and the distal apexes define a cylindrical surface. A pair of the anchor pins is located on the struts adjacent each of the proximal apexes, the anchor pins extending inwards (relative to the curve of the proximal apexes) from inside surfaces of the struts and protruding from the struts radially outward from the cylindrical (outer circumferential) surface.

By locating the anchor pins inwards, the delivery profile, sometimes called crimped profile, of the proximal anchor stent ring is minimized.

In accordance with another example, a method of deploying an endoprosthesis includes radially constraining a proximal anchor stent ring of the endoprosthesis in an annular space between a sleeve of a tip and a spindle, the spindle having distal spindle pins extending into proximal spindle pin catches of the proximal anchor stent ring. A distal anchor stent ring of the endoprosthesis is radially constrained in an annular space between a middle member sleeve and the spindle, the spindle comprising proximal spindle pins extending into distal spindle pin catches of the distal anchor stent ring. Further, a graft material of the endoprosthesis is radially constrained in a primary sheath, the graft material being attached to the proximal anchor stent ring and the distal anchor stent ring.

The primary sheath is retracted to deploy at least a portion of the endoprosthesis. The tip is advanced to deploy the proximal anchor stent ring. Further, the middle member sleeve is retracted to deploy the distal anchor stent ring.

By providing a proximal capture and release mechanism for controlled deployment of proximal anchor stent ring and a distal capture and release mechanism for controlled deployment of distal anchor stent ring, deployment of the endoprosthesis occurs in three distinct and interchangeable operations. This provides maximum control in the deployment of the endoprosthesis.

According to another aspect, a delivery system comprises a spindle comprising a spindle body; and distal spindle pins extending radially outward from said spindle body; and proximal spindle pins extending radially outward from said spindle body; a tip comprising a sleeve, said distal spindle pins extending from said spindle body toward said sleeve; a middle member comprising a middle member sleeve, said proximal spindle pins extending from said spindle body toward said middle member sleeve; and an endoprosthesis comprising: a graft material; a proximal anchor stent ring attached to said graft material, said proximal anchor stent ring comprising proximal spindle pin catches, said distal spindle pins extending into said proximal spindle pin catches; and a distal anchor stent ring attached to said graft material, said distal anchor stent ring comprising distal spindle pin catches, said proximal spindle pins extending into said distal spindle pin catches.

In another aspect, a delivery system comprises an outer member; a spindle on a distal end of said outer member, said spindle comprising: a spindle body; and distal spindle pins extending radially outward from said spindle body; a tip comprising a sleeve, said distal spindle pins extending from said spindle body toward said sleeve; a middle member comprising a middle member sleeve; and an endoprosthesis comprising proximal spindle pin catches at a proximal end of said endoprosthesis and a distal anchor stent ring at a distal end of said endoprosthesis, said distal spindle pins extending into said proximal spindle pin catches, said distal anchor stent ring being restrained within said middle member sleeve. In typical embodiments thereof, an annular space exists between said outer member and said middle member sleeve. Typically, the distal anchor stent ring is restrained within this annular space.

In even another aspect, a delivery system comprises an outer member; a spindle on a distal end of said outer member, said spindle comprising: a spindle body; and distal spindle pins extending radially outward from said spindle body; a tip comprising a sleeve, said distal spindle pins extending from said spindle body toward said sleeve; a middle member comprising a middle member sleeve; and an endoprosthesis comprising: a graft material; a proximal anchor stent ring attached to said graft material, said proximal anchor stent ring comprising proximal spindle pin catches, said distal spindle pins extending into said proximal spindle pin catches; and a distal anchor stent ring attached to said graft material, said distal anchor stent ring being restrained within said middle member sleeve.

These and other features according to embodiments and aspects of the present invention will be more readily apparent from the detailed description set forth below taken in conjunction with the accompanying drawings. Furthermore, it will be understood by those skilled in the art that features of one embodiment or aspect may be readily employed in or replaced with another feature of like functionality used in another embodiment or aspect described herein. In particular, spindle pins may be omitted at one or both ends of the endoprosthesis in some configurations.

### BRIEF DESCRIPTION OF THE DRAWINGS

FIG. 1 is a partial cross-sectional view of a stent-graft delivery system without a stent-graft and outer sheath in accordance with one embodiment;

FIG. 2 is a partial cross-sectional view of the stent-graft delivery system of FIG. 1 including a stent-graft located within a retractable primary sheath in a pre-deployment un-retracted position;

FIG. 3 is a partial cross-sectional view of the stent-graft delivery system of FIG. 2 with the retractable primary sheath partially retracted;

FIG.4 is a partial cross-sectional view of the stent-graft delivery system of FIG. 3 after deployment of a proximal anchor stent ring of the stent-graft;

FIG. 5 is a perspective view of an expanded stent-graft similar to the stent-graft of FIGS. 2, 3 and 4;

FIG. 6 is perspective view of an expanded proximal anchor stent ring similar to a proximal anchor stent ring of the stent-graft of FIG. 5;

FIG. 7 is a top view of the proximal anchor stent ring of FIG. 6;

FIG. 8 is a cross-sectional view of the proximal anchor stent ring along the line VIII-VIII of FIG. 7;

FIG. 9 is an enlarged view of a region IX of the proximal anchor stent ring of FIG. 8;

FIG. 10 is a side view of the region of the proximal anchor stent ring of FIG. 9;

FIG. 11 is a cross-sectional view of the proximal anchor stent ring along the line XI-XI of FIG. 7;

FIG. 12 is an a flattened pattern of the as cut proximal anchor stent ring of FIGS. 6-11;

FIG. 13 is a partial cross-sectional view of a proximal anchor stent ring anchored in a vessel wall in accordance with one embodiment;

FIG. 14 is an enlarged partially cutaway view of a stent-graft delivery system in accordance with another embodiment;

FIG. 15 is a cross-sectional view of a stent-graft delivery system in accordance with another embodiment;

FIG. 16 is a cross-sectional view of the stent-graft delivery system of FIG. 15 at a further stage during deployment of a stent-graft;

FIG. 17 is a cross-sectional view of the stent-graft delivery system of FIG. 16 at a final stage during deployment of the stent-graft;

FIG. 18 is a handle of a stent-graft delivery system in accordance with one embodiment;

FIG. 19 is a partial cross-sectional view of a stent-graft delivery system in accordance with another embodiment;

FIG. 20 is an enlarged side view of the region XX of the stent-graft delivery system of FIG. 19;

FIG. 21 is a handle of a stent-graft delivery system in accordance with one embodiment;

FIG. 22 is the stent-graft delivery system of FIGS. 19, 20 including a handle;

FIG. 23 is a partial cross-sectional view of the stent-graft delivery system of FIG. 22;

FIG. 24 is a side view of a middle member of the stent-graft delivery system of FIGS. 22 and 23;

FIG. 25 is a side view of a portion of a housing of the stent-graft delivery system of FIGS. 22 and 23;

FIGS. 26, 27, 28 are side views of the stent-graft delivery system of FIGS. 22 and 23 at various stages during deployment of a stent-graft; and

FIG. 29 is an enlarged side view of a region of a stent-graft delivery system in accordance with one embodiment.

In the following description, the same or similar elements are labeled with the same or similar reference numbers.

### DETAILED DESCRIPTION

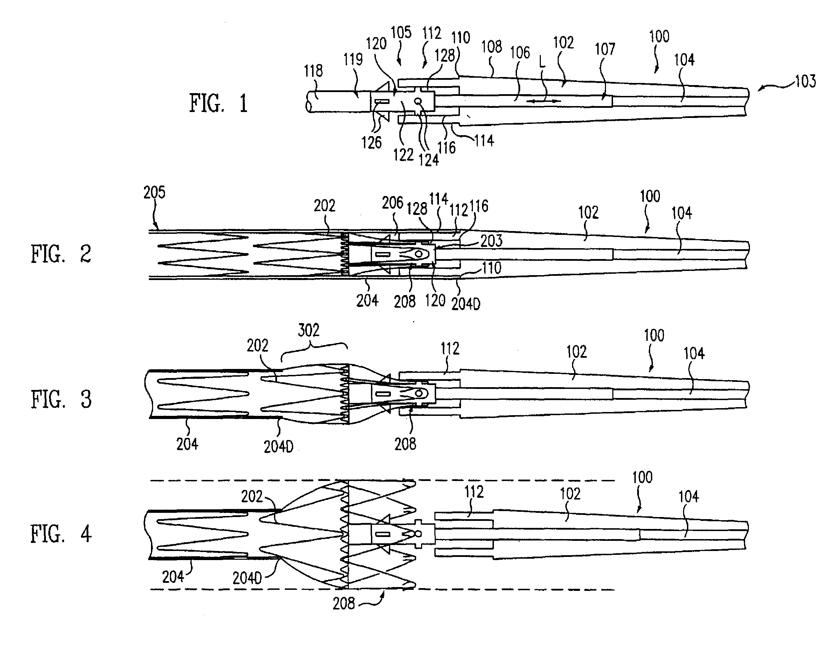

FIG. 1 is a partial cross-sectional view of a stent-graft delivery system 100 without a stent-graft and outer sheath in accordance with one embodiment. Stent-graft delivery system 100 includes a tapered tip 102 that is flexible and able to provide trackability in tight and tortuous vessels. Tapered tip 102 includes a guidewire lumen 104 therein for connecting to adjacent members and allowing passage of a guidewire through tapered tip 102. Other tip shapes such as bullet-shaped tips could also be used.

An inner tube 106 defines a lumen, e.g., a guide wire lumen, therein. A distal end 107 of inner tube 106 is located within and secured to tapered tip 102, i.e., tapered tip 102 is mounted on inner tube 106. As shown in FIG. 1, the lumen of inner tube 106 is in fluid communication with guidewire lumen 104 of tapered tip 102 such that a guide wire can be passed through inner tube 106 and out distal end 107, through guidewire lumen 104 of tapered tip 102, and out a distal end 103 of tapered tip 102.

Tapered tip 102 includes a tapered outer surface 108 that gradually increases in diameter. More particularly, tapered outer surface 108 has a minimum diameter at distal end 103 and gradually increases in diameter proximally, i.e., in the direction of the operator (or handle of stent-graft delivery system 100), from distal end 103.

Tapered outer surface 108 extends proximally to a primary sheath abutment surface (shoulder) 110 of tapered tip 102. Primary sheath abutment surface 110 is an annular ring perpendicular to a longitudinal axis L of stent-graft delivery system 100.

Tapered tip 102 further includes a (tip) sleeve 112 extending proximally from primary sheath abutment surface 110. Generally, sleeve 112 is at a proximal end 105 of tapered tip 102. Sleeve 112 is a hollow cylindrical tube extending proximally and longitudinally from primary sheath abutment surface 110. Sleeve 112 includes an outer cylindrical surface 114 and an inner cylindrical surface 116.

Stent-graft delivery system 100 further includes an outer tube 118 having a spindle 120 located at and fixed to a distal end 119 of outer tube 118. Spindle 120 includes a spindle body 122 having a cylindrical outer surface, a plurality of spindle pins 124 protruding radially outward from spindle body 122, and a plurality of primary sheath guides 126 protruding radially outward from spindle body 122. Primary sheath guides 126 guide the primary sheath into position over (tip) sleeve 112 (see FIG. 2 for example).

As illustrated in FIG. 1, spindle 120 is configured to slip inside of sleeve 112 such that spindle pins 124 are directly adjacent to, or contact, inner cylindrical surface 116 of sleeve 112. Spindle pins 124 extend from spindle body 122 towards and to sleeve 112. Generally, the diameter to which spindle pins 124 extend from spindle body 112 is approximately equal to, or slightly less than, the diameter of inner cylindrical surface 116 of sleeve 112 allowing spindle pins 124 to snugly fit inside of sleeve 112. An annular space 128 exists between inner cylindrical surface 116 and spindle body 122.

Inner tube 106 is within and extends through outer tube 118 and spindle 120. Inner tube 106 and thus tapered tip 102 is moved along longitudinal axis L (longitudinally moved) relative to outer tube 118 and thus spindle 120 to release the proximal end of a stent-graft as discussed further below. The term "stent-graft" used herein should be understood to include stent-grafts and other forms of endoprosthesis.

FIG. 2 is a partial cross-sectional view of the stent-graft delivery system 100 of FIG. 1 including a stent-graft 202 located within a retractable primary sheath 204 in a pre-deployment un-retracted position.

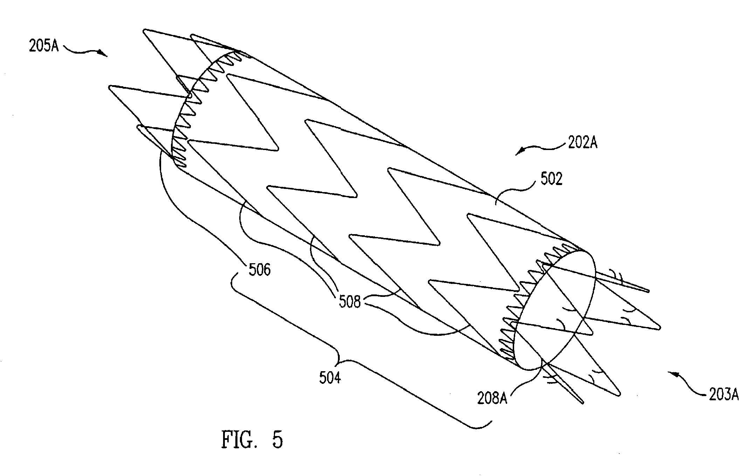

Primary sheath 204 is a hollow tube and defines a lumen 206 therein through which outer tube 118 and inner tube 106 extend. Primary sheath 204 is in a pre-deployment un-retracted position in FIG. 2. Primary sheath 204 is moved proximally along longitudinal axis L, sometimes called retracted, relative to outer tube 118/spindle 120 and thus stent-graft 202 to deploy a portion of stent-graft 202 as discussed further below. In one embodiment, stent-graft 202 is a self-expanding stent-graft such that stent-graft 202 self-expands upon being released from its radially constrained position. In accordance with this example, stent-graft 202 includes a graft material and support structures attached to the graft material as discussed in greater detail below with reference to FIG. 5. Stent-graft 202 includes a proximal end 203 and a distal end 205.

As shown in FIG. 2, stent-graft 202 is in a radially constrained configuration over outer tube 118 and spindle 120. Stent-graft 202 is located within and radially compressed by primary sheath 204. Further, a proximal anchor stent ring 208, sometimes called the proximal tip, of stent-graft 202 is radially constrained and held in position in annular space 128 between spindle body 122 and inner cylindrical surface 116 of sleeve 112. Proximal anchor stent ring 208 is at proximal end 203 of stent-graft 202.

Generally, the graft material of stent-graft 202 is radially constrained by primary sheath 204 and the proximal portion of proximal anchor stent ring 208 is radially constrained by sleeve 112 allowing sequential and independent deployment of the graft material and proximal anchor stent ring 208 of stent-graft 202.

Primary sheath 204 includes a distal end 204D adjacent to or in abutting contact with primary sheath abutment surface 110 of tapered tip 102. Distal end 204D fits snugly around sleeve 112 and in one example lightly presses radially inward on outer cylindrical surface 114 of sleeve 112.

FIG. 3 is a partial cross-sectional view of the stent-graft delivery system 100 of FIG. 2 with retractable primary sheath 204 partially retracted. Referring now to FIG. 3, primary sheath 204 is partially retracted such that distal end 204D is spaced apart from tapered tip 102. Further, due to the retraction of primary sheath 204, a proximal portion 302 of stent-graft 202 is exposed and partially deployed. Proximal portion 302 is a portion of stent-graft 202 distal to proximal anchor stent ring 208 but proximal to the remaining portion of stent-graft 202.

As proximal portion 302 is only partially deployed and a portion of proximal anchor stent ring 208 is radially constrained and un-deployed, stent-graft 202 can be repositioned in the event that the initial positioning of stent-graft 202 is less than desirable. More particularly, to reposition stent-graft 202, the retraction of primary sheath 204 is halted. Stent-graft delivery system 100 is then moved to reposition stent-graft 202, for example, stent-graft 202 is rotated or moved proximally or distally without a substantial risk of damaging the wall of the vessel in which stent-graft 202 is being deployed.

Further, as proximal end 203 of stent-graft 202 is secured fixing proximal end 203 of stent-graft 202 and keeping it in tension as primary sheath 204 is retracted and, in one example, distal end 205 is free to move within primary sheath 204, bunching of stent-graft 202 during retraction of primary sheath 204 is avoided. By avoiding bunching, frictional drag of stent-graft 202 on primary sheath 204 during retraction is minimized thus facilitating smooth and easy retraction of primary sheath 204.

Once stent-graft 202 is properly positioned, proximal anchor stent ring 208 is released and deployed securing stent-graft 202 in position within the vessel as discussed in greater detail below.

FIG. 4 is a partial cross-sectional view of the stent-graft delivery system 100 of FIG. 3 after deployment of proximal anchor stent ring 208 of stent-graft 202.
Referring now to FIG. 4, tapered tip 102 is advanced relative to spindle 120 to expose the proximal end of proximal anchor stent ring 208. Upon being released from sleeve 112 of tapered tip 102, the proximal end of proximal anchor stent ring 208 self-expands into the wall of the vessel in which stent-graft 202 is being deployed.

As set forth below, proximal anchor stent ring 208 includes anchor pins which penetrate into the surrounding vessel wall thus anchoring proximal anchor stent ring 208 to the wall of the vessel. Accordingly, after deployment and anchoring of proximal anchor stent ring 208 to the vessel wall, primary sheath 204 is fully retracted to fully deploy stent-graft 202 without migration.

However, in another example, primary sheath 204 is fully retracted prior to release of proximal anchor stent ring 208. To illustrate, instead of being partially retracted at the stage of deployment illustrated in FIG. 3, primary sheath 204 is fully retracted while the proximal end of proximal anchor stent ring 208 is still radially constrained.

Further, stent-graft 202 is set forth above as being a self-expanding stent. In accordance with another embodiment, instead of being a self-expanding stent-graft, stent-graft delivery system 100 includes an expansion member, e.g., a balloon, which is expanded to expand and deploy the stent-graft.

FIG. 5 is a perspective view of an expanded stent-graft 202A similar to stent-graft 202 of FIGS. 2, 3 and 4. Referring now to FIG. 5, stent-graft 202A includes a graft material 502, e.g., formed of polyester or Dacron material, and a plurality of resilient self-expanding support structures 504, e.g., formed of super elastic self-expanding memory material such as Nitinol, including a proximal anchor stent ring 208A at a proximal end 203A, a distal stent ring 506 at a distal end 205A, and stent rings 508 between proximal anchor stent ring 208A and distal stent ring 506. Support structures 504 are attached to graft material 502, e.g., by sutures, adhesive, or other means.

Typically, stent-graft 202A is deployed such that graft material 502 spans, sometimes called excludes, a diseased portion of the vessel, e.g., an aneurysm. Further, proximal anchor stent ring 208A, e.g., a suprarenal stent structure, is typically engaged with a healthy portion of the vessel adjacent the diseased portion, the healthy portion having stronger tissue than the diseased portion. By forming proximal anchor stent ring 208A with anchor pins as discussed below, the anchor pins penetrate (land) into the vessel wall of the healthy tissue thus anchoring proximal anchor stent ring 208 to strong tissue.

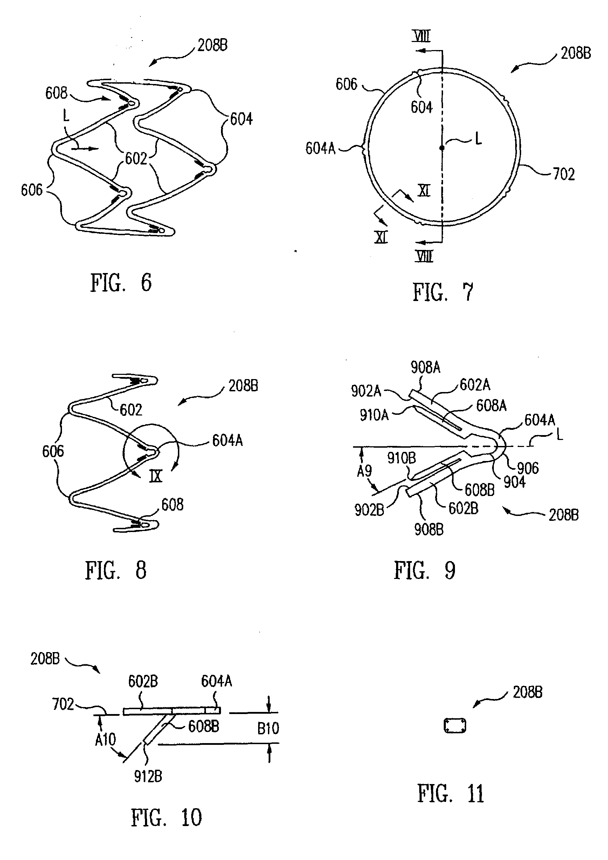

FIG. 6 is perspective view of an expanded proximal anchor stent ring 208B similar to proximal anchor stent ring 208A of stent-graft 202A of FIG. 5. FIG. 7 is a top view of proximal anchor stent ring 208B of FIG. 6. FIG. 8 is a cross-sectional view of proximal anchor stent ring 208B along the line VIII-Vill of FIG. 7. FIG. 9 is an enlarged view of a region IX of proximal anchor stent ring 208B of FIG. 8. FIG. 10 is a side view of the region of proximal anchor stent ring 208B of FIG. 9. FIG. 11 is a cross-sectional view of proximal anchor stent ring 208B along the line XI-XI of FIG. 7.

Referring now to FIGS. 6, 7, 8, 9, 10, and 11 together, proximal anchor stent ring 208B includes a zigzag pattern of struts 602 alternating between proximal apexes 604 and distal apexes 606. Illustratively, proximal anchor stent ring 208B is laser cut from a one-piece material such as a tube. After being cut, proximal anchor stent ring 208B is sequentially expanded, e.g., using a mandrel, and heat set, into its final expanded configuration as those of skill in the art will understand in light of this disclosure. In one example, the mandrel includes protruding features which facilitate heat setting of the anchor pins in position.

Distal apexes 606 are attached to the graft material of the stent-graft, e.g., see graft material 502 of FIG. 5. Proximal anchor stent ring 208B further includes anchor pins 608.

More particularly, a pair of anchor pins 608 is located on struts 602 adjacent each proximal apex 604. By locating anchor pins 608 adjacent proximal apexes 604, the effect on the flexibility of proximal anchor stent ring 208B by anchor pins 608 is minimal. Further, as proximal anchor stent ring 208B is integral in one example, i.e., is a single piece laser cut from a tube and not a plurality of separate pieces attached together, anchor pins 608 are durable, e.g., are not likely to break off or otherwise fail.

Referring now to FIG. 9, a first proximal apex 604A of the plurality of proximal apexes 604 is illustrated. First and second struts 602A, 602B of the plurality of struts 602 extends distally from proximal apex 604A. A first anchor pin 608A of the plurality of anchor pins 608 extends from strut 602A adjacent proximal apex 604A. Similarly, a second anchor pin 608B of the plurality of anchor pins 608 extends from strut 602B adjacent proximal apex 604A.

In one embodiment, the angle of anchor pins 608 from the vertical (horizontal in the view of FIG. 9) is in the range of 0° to 50°, e.g., feature A9 is in the range of 0° to 50° and in one example is 45°. By forming anchor pins 608 at an angle in the range of 0° to 50° from the vertical, anchor pins 608 are in line with any force for migration, e.g., force in the distal direction (force in the left direction in the view of FIG. 9). In one embodiment, the vertical is parallel to the longitudinal axis L of proximal anchor stent ring 208B.

Anchor pins 608A, 608B extend from the inside surfaces 902A, 902B of struts 602A, 602B, respectively. As used herein, the inside and outside surfaces of struts 602 are defined relative to proximal apexes 604. More particularly, the inside surface of a strut 602 is the surface that correlates and extends smoothly from the inside radial surface of the curved apex, i.e., the curvature of proximal apexes 604. Conversely, the outside surface of a strut 602 correlates to the outside radial surface of proximal apexes 604. Generally, the outside surfaces of struts 602 are proximal to the inside surfaces of struts 602.

To illustrate, proximal apex 604A includes an intrados (the interior curve of an arch) surface 904 and an extrados (the exterior curve of an arch) surface 906, extrados surface 906 having a greater radius then intrados surface 904. Extrados surface 906 is continuous with outside surfaces 908A, 908B of struts 602A, 602B, respectively. Similarly, intrados surface 904 is continuous with inside surfaces 902A, 902B of struts 602A, 602B, respectively. Stated another way, anchor pins 608A, 608B extend inwards from struts 602A, 602B, respectively.

Generally, anchor pins 608 are located inwards of struts 602. By locating anchor pins 608 inwards, the delivery profile, sometimes called crimped profile, of proximal anchor stent ring 208B is minimized in contrast to a configuration where anchor pins are located outward and space must be allocated to accommodate the anchor pins.

In accordance with this example, anchor pins 608 include distal tips, e.g., sharp points, which facilitate penetration of anchor pins 608 into the wall of the vessel in which the stent-graft is deployed. To illustrate, paying particular attention to FIG. 9, anchor pins 608A, 608B include distal tips 910A, 910B, respectively.

Further, anchor pins 608A, 608B protrude radially outward from the cylindrical surface (plane) defined by the zigzag pattern of struts 602 alternating between proximal apexes 604 and distal apexes 606. Generally, anchor pins 608A, 608B protrude radially outward from proximal anchor stent ring 208B.

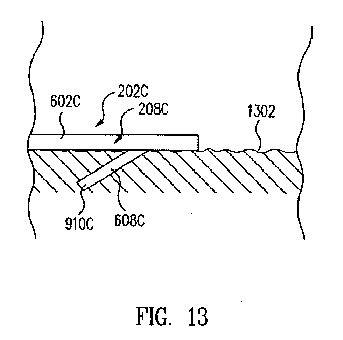

Paying particular attention now to FIGS. 7 and 10, struts 602, proximal apexes 604, and distal apexes 606 define a cylindrical surface 702. Anchor pins 608 protrude from struts 602 radially outward from (imaginary) cylindrical surface 702. As discussed in greater detail below with reference to FIG. 13, by protruding radially outwards from proximal anchor stent ring 208B, anchor pins 608 penetrate into the vessel wall thus anchoring proximal anchor stent ring 208B and the corresponding stent-graft to the vessel wall.

Illustratively, anchor pins 608 protrude radially outward (the radial distance from the imaginary cylindrical surface 702 in contrast to the length of anchor pins 608) from struts 602 a distance in the range of one millimeter to three millimeters, i.e., feature B10 of FIG. 10 is 3 mm, in the range of 1 mm to 3 mm in one example, and in the range of 2 mm to 3mm in another example. Further, feature A10, i.e., the angle of intersection between anchor pins 608 and struts 602 is 45° or in the range of 30° to 75° in one example. By forming the angle of intersection in the range of 30° to 75°, any force in the distal direction on proximal anchor stent ring 208B (left in the view of FIG. 10) causes anchor pins 608 to penetrate (dig) deeper into the vessel wall thus pulling struts 602 and proximal apexes 604 tighter to the vessel wall effectively locking proximal anchor stent ring 208B to the vessel wall.

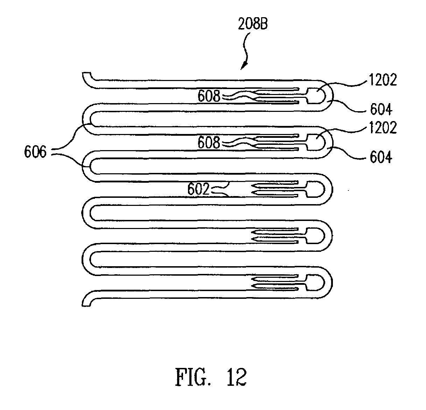

FIG. 12 is an as cut flat pattern of proximal anchor stent ring 208B of FIGS. 6-11. Referring now to FIG. 12, proximal anchor stent ring 208B is illustrated in its unexpanded configuration, sometimes called delivery profile. In its unexpanded configuration, proximal apexes 604 and anchor pins 608 define spindle pin catches 1202.

Spindle pin catches 1202 are pockets, sometimes called openings or holes, in which the spindle pins of the stent-graft delivery system are located to radially constrain proximal anchor stent ring 208B in its unexpanded configuration (crimped profile) prior to deployment as discussed in greater detail below. Generally, anchor pins 608 are positioned slightly distal from proximal apexes 604 to leave room for the spindle pins.

Although proximal anchor stent ring 208B is illustrated as having five proximal apexes 604 and five distal apexes 606, sometimes called a five apex proximal anchor stent ring, in other examples, a proximal anchor stent ring has more or less than five proximal apexes and five distal apexes, e.g., four or six of each, sometimes called a four or six apex proximal anchor stent ring.

FIG. 13 is a partial cross-sectional view of a proximal anchor stent ring 208C of a stent-graft 202C anchored in a vessel wall 1302 in accordance with one embodiment. Referring now to FIG. 13, an anchor pin 608C is extending radially outward from a strut 602C and penetrating into vessel wall 1302. Distal tip 910C of anchor pin 608C facilitates penetration of anchor pin 608C into vessel wall 1302, e.g., healthy tissue. Accordingly, proximal anchor stent ring 208C is anchored to vessel wall 1302 preventing migration of stent-graft 202C in the distal direction, i.e., prevents motion of stent-graft 202C towards the left in the view of FIG. 13.

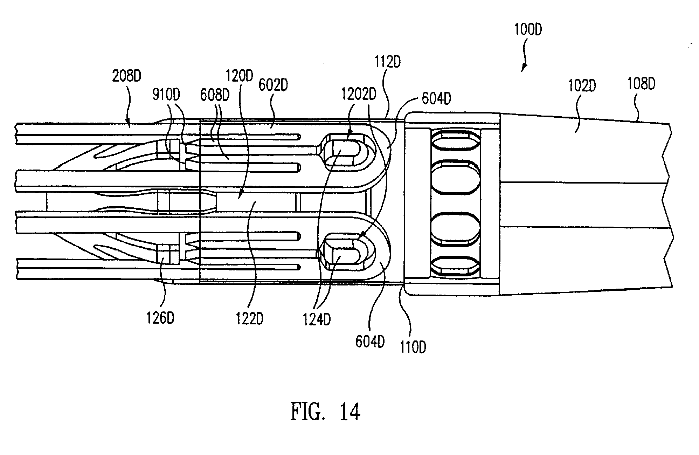

FIG. 14 is an enlarged partially cutaway view of a stent-graft delivery system 100D in accordance with another embodiment. Referring now to FIG. 14, a proximal portion of proximal anchor stent ring 208D is restrained within a sleeve 112D of a tapered tip 102D. Sleeve 112D is illustrated as a transparent sleeve in FIG. 14 to illustrate features within sleeve 112D. However, in other examples, sleeve 112D is opaque. Illustratively, sleeve 112D is stainless steel, Nitinol, MP35N alloy, or a polymer.

Spindle pins 124D of a spindle 120D extend into and are located within spindle pin catches 1202D of proximal anchor stent ring 208D. Accordingly, the proximal end of proximal anchor stent ring 208D is locked around spindle pins 124D and between sleeve 112D and a spindle body 122D. Illustratively, spindle 120D is stainless steel, Nitinol, MP35N alloy, or a polymer.

Further, sleeve 112D holds anchor pins 608D down (radially inward) thus providing a minimal delivery profile for proximal anchor stent ring 208D. Generally, sleeve 112D holds anchor pins 608D bent in a lower profile.

Sleeve 112D does not cover (exposes) distal tips 910D of anchor pins 608D. Stated another way, sleeve 112D extends distally only partially over anchor pins 608D. This prevents distal tips 910D, e.g., sharp tips, from engaging (digging into, scratching, gouging) sleeve 112D. This minimizes the deployment force necessary to advance sleeve 112D relative to proximal anchor stent ring 208D.

Tapered outer surface 108D, primary sheath abutment surface 110D, primary sheath guides 126D, struts 602D, proximal apexes 604D are similar to tapered outer surface 108, primary sheath abutment surface 110, primary sheath guides 126, struts 602, proximal apexes 604 as discussed above, respectively, and so the description thereof is not repeated here.

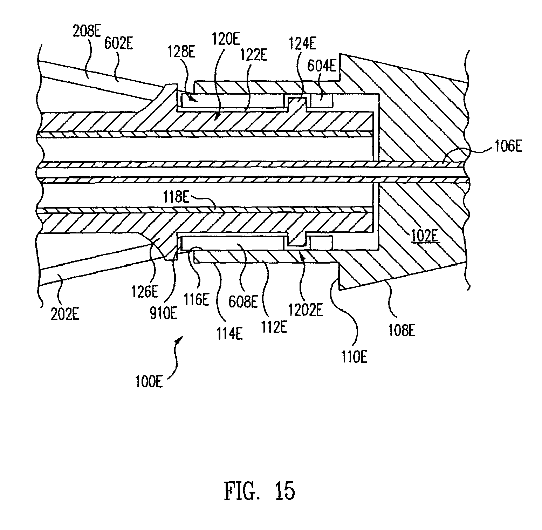

FIG. 15 is a cross-sectional view of a stent-graft delivery system 100E in accordance with another embodiment. FIG. 15 corresponds to the stage similar to that illustrated in FIG. 3 of deployment of a stent-graft 202E, i.e., after at least partial retraction of the primary sheath.

Referring now to FIG. 15, stent-graft delivery system 100E includes a tapered tip 102E, an inner tube 106E, a tapered outer surface 108E, a primary sheath abutment surface 110E, a sleeve 112E, an outer cylindrical surface 114E, an inner cylindrical surface 116E, an outer tube 118E, a spindle 120E, a spindle body 122E, spindle pins 124E, primary sheath guides 126E, an annular space 128E similar to tapered tip 102, inner tube 106, tapered outer surface 108, primary sheath abutment surface 110, sleeve 112, outer cylindrical surface 114, inner cylindrical surface 116, outer tube 118, spindle 120, spindle body 122, spindle pins 124, primary sheath guides 126, annular space 128 of stent-graft delivery system 100 of FIGS. 1-4, respectively.

Further, stent-graft 202E includes a proximal anchor stent ring 208E including struts 602E, proximal apexes 604E, anchor pins 608E, distal tips 910E, and spindle pin catches 1202E similar to proximal anchor stent ring 208B including struts 602, proximal apexes 604, anchor pins 608, distal tips 910, and spindle pin catches 1202 of proximal anchor stent ring 208B of FIGS. 6-12, respectively.

As shown in FIG. 15, the proximal end of proximal anchor stent ring 208E is restrained within sleeve 112E of tapered tip 102E. Spindle pins 124E of spindle 120E are located within spindle pin catches 1202E of proximal anchor stent ring 208E. Accordingly, proximal anchor stent ring 208E is locked around spindle pins 124E and between sleeve 112E and a spindle body 122E.

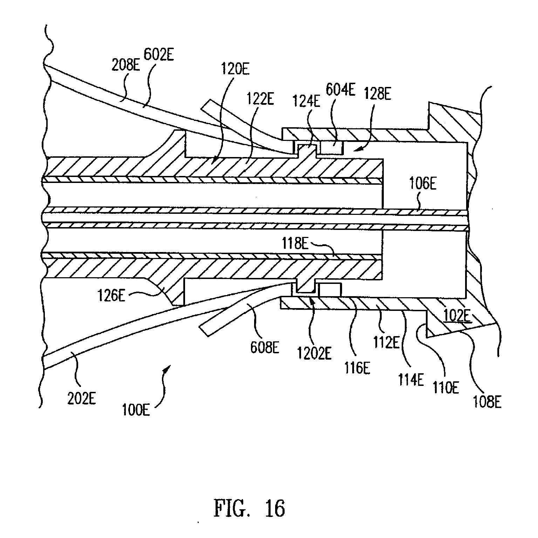

FIG. 16 is a cross-sectional view of stent-graft delivery system 100E of FIG. 15 at a further stage during deployment of stent-graft 202E. Referring now to FIG. 16, tapered tip 102E and thus sleeve 112E are advanced relative to spindle 120E. However, as spindle pins 124E are still located within sleeve 112E, the proximal end of proximal anchor stent ring 208E continues to be locked around spindle pins 124E and between sleeve 112E and spindle body 122E.

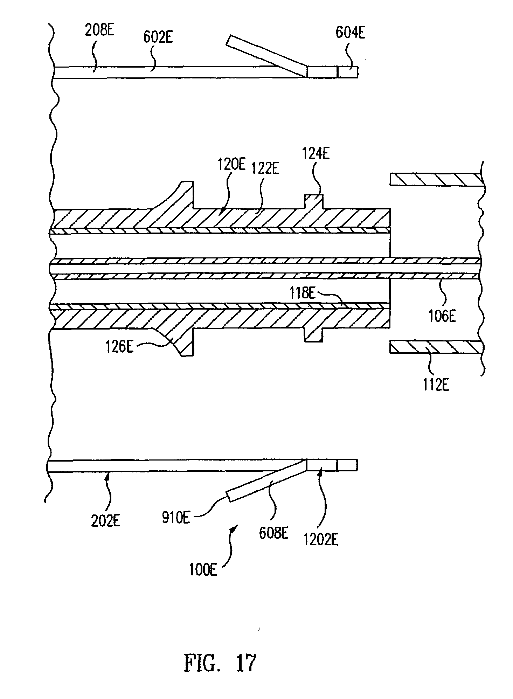

FIG. 17 is a cross-sectional view of stent-graft delivery system 100E of FIG. 16 at a final stage during deployment of stent-graft 202E. FIG. 17 corresponds to the stage of deployment of stent-graft 202E similar to that illustrated in FIG. 4, i.e., after the proximal end of the proximal anchor stent ring has been deployed.

Referring now to FIG. 17, tapered tip 102E and thus sleeve 112E are advanced relative to spindle 120E such that sleeve 112E uncovers and exposes spindle pins 124E and proximal apexes 604E of proximal anchor stent ring 208E. Upon being released, proximal anchor stent ring 208E self-expands and anchors into the vessel wall, e.g., in a manner similar to that discussed above regarding FIG. 13.

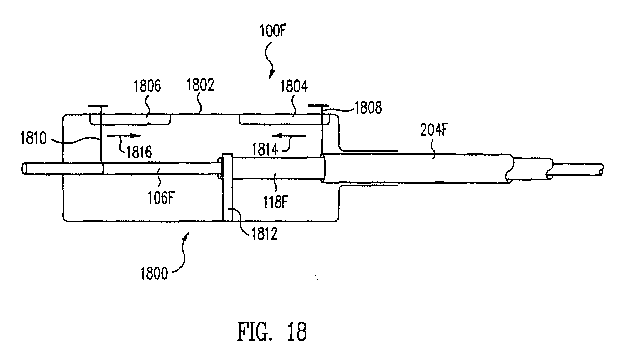

FIG. 18 is a handle 1800 of a stent-graft delivery system 100F in accordance with one embodiment. Handle 1800 includes a housing 1802 having a primary sheath retraction slot 1804 and an inner tube advancement slot 1806. A primary sheath actuation member 1808, sometimes called a thumb slider, extends from a primary sheath 204F and through primary sheath retraction slot 1804. Similarly, an inner tube actuation member 1810, sometimes called a thumb slider, extends from an inner tube 106F and through inner tube advancement slot 1806. Further, an outer tube 118F is mounted to housing 1802 by an outer tube support 1812.

To retract primary sheath 204F relative to outer tube 118F, primary sheath actuation member 1808 is moved (retracted), e.g., by the physician, in the direction of arrow 1814. To advance inner tube 106F relative to outer tube 118F, inner tube actuation member 1810 is moved (advanced), e.g., by the physician, in the direction of arrow 1816. Illustratively, inner tube 106F and outer tube 118F are stainless steel, Nitinol, MP35N alloy, or a braided polymer.

Although one example of a handle is set forth in FIG. 18, in light of this disclosure, those of skill in the art will understand that other handles can be used. Illustratively, handles having ratcheting mechanisms, threaded mechanisms, or other mechanisms to retract the primary sheath and advance the inner tube relative to the outer tube are used.

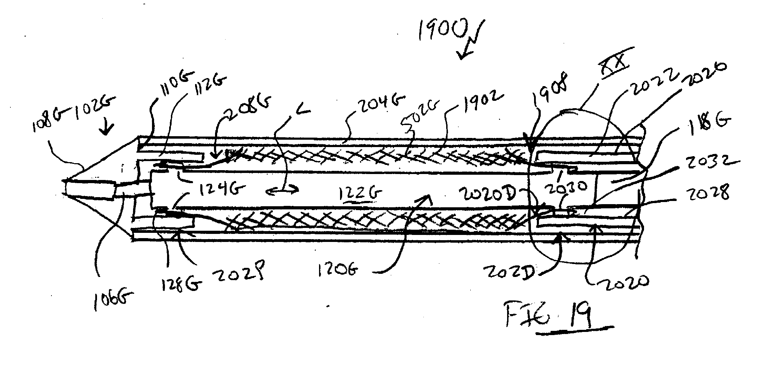

FIG. 19 is a partial cross-sectional view of a stent-graft delivery system 1900 in accordance with another embodiment. Referring now to FIG. 19, stent-graft delivery system 1900 includes a tapered tip 102G, an inner tube 106G, a tapered outer surface 108G, a primary sheath abutment surface 110G, a sleeve 112G, an outer tube 118G, a spindle 120G, a spindle body 122G, spindle pins 124G, sometimes called distal spindle pins, an annular space 128G, and a primary sheath 204G similar to tapered tip 102, inner tube 106, tapered outer surface 108, primary sheath abutment surface 110, sleeve 112, outer tube 118, spindle 120, spindle body 122, spindle pins 124, annular space 128, and primary sheath 204 of stent-graft delivery system 100 of FIGS. 1-4, respectively.

Further, stent-graft delivery system 1900 includes a stent-graft 1902, e.g., an abdominal or thoracic stent-graft. Stent-graft 1902 includes a proximal anchor stent ring 208G including struts, proximal apexes, anchor pins, distal tips, and proximal spindle pin catches similar to proximal anchor stent ring 208B including struts 602, proximal apexes 604, anchor pins 608, distal tips 910, and spindle pin catches 1202 of proximal anchor stent ring 208B of FIGS. 6-12, respectively. Further, stent-graft 1902 includes a graft material 502G similar to graft material 502 of stent-graft 202A of FIG. 5. Generally, spindle 120G and sleeve 112G form a proximal capture and release mechanism for proximal anchor stent ring 208G.

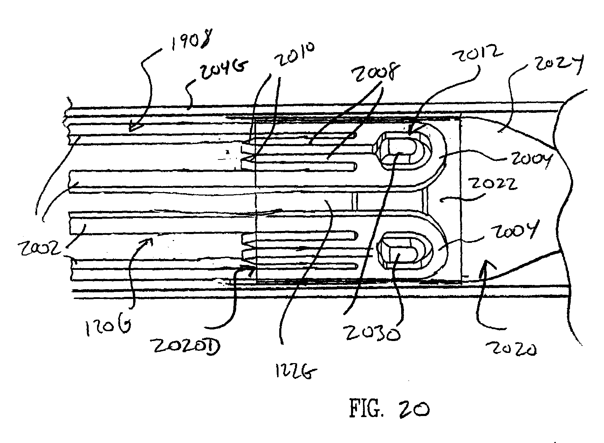

FIG. 20 is a side perspective view of the region XX of stent-graft delivery system 1900 of FIG. 19. Referring now to FIGS. 19 and 20 together, stent-graft 1902 further includes distal anchor stent ring 1908 including struts 2002, distal apexes 2004, anchor pins 2008, proximal tips 2010, and spindle pin catches 2012 similar to proximal anchor stent ring 208B including struts 602, proximal apexes 604, anchor pins 608, distal tips 910, and spindle pin catches 1202 of proximal anchor stent ring 208B of FIGS. 6-12, respectively.

Generally, proximal anchor stent ring 208G is located at the proximal end 202P of stent-graft 202G and distal anchor stent ring 1908 is located at the distal end 202D of stent-graft 202G. Proximal anchor stent ring 208G and distal anchor stent ring 1908 are attached to graft material 502G of stent-graft 1902. Distal anchor stent ring 1908 is similar or identical to proximal anchor stent ring 208G except that the orientation is reversed. More particularly, spindle pin catches 2012 are located at the distal end of distal anchor stent ring 1908 and anchor pins 2008 point proximally away from spindle pin catches 2012 towards proximal anchor stent ring 208G.

Stent-graft delivery system 1900 further includes a middle member 2020 having a middle member sleeve 2022 extending distally from a middle member tube 2024 of middle member 2020. Generally, middle member sleeve 2022 is at a distal end 2020D of middle member 2020. Middle member sleeve 2022 is a hollow cylindrical tube extending distally and longitudinally from middle member tube 2024. Middle member sleeve 2022 includes an outer cylindrical surface 2026 and an inner cylindrical surface 2028. Middle member sleeve 2022 is sometimes called a stent stop or a distal stent cup/sleeve.

Stent-graft delivery system 1900 further includes outer tube 118G having spindle 120G located at and fixed to the distal end of outer tube 118G. Spindle 120G includes spindle body 122G having a cylindrical outer surface, distal spindle pins 124G protruding radially outward from spindle body 122G, and a plurality of proximal spindle pins 2030 protruding radially outward from spindle body 122G.

As illustrated in FIGS. 19 and 20, spindle 120G is configured to slip inside of middle member sleeve 2022 such that proximal spindle pins 2030 are directly adjacent to, or contact, inner cylindrical surface 2028 of middle member sleeve 2022. Proximal spindle pins 2030 extend from spindle body 122G towards and to middle member sleeve 2022. Generally, the diameter to which proximal spindle pins 2030 extend from spindle body 122G is approximately equal to, or slightly less than, the diameter of inner cylindrical surface 2028 of middle member sleeve 2022 allowing proximal spindle pins 2030 to snugly fit inside of middle member sleeve 2022. An annular space 2032 exists between inner cylindrical surface 2028 and spindle body 122G.

Middle member 2020 is a hollow tube and defines a lumen therein through which outer tube 118G and inner tube 106G extend. Middle member 2020 and thus middle member sleeve 2022 is moved along longitudinal axis L (longitudinally moved) relative to outer tube 118G and thus spindle 120G to release distal end 202D (distal anchor stent ring 1908) of stent-graft 1902 as discussed further below. Primary sheath 204G is a hollow tube and defines a lumen therein through which middle member 2020, outer tube 118G and inner tube 106G extend.

Distal anchor stent ring 1908 is illustrated in its unexpanded configuration, sometimes called delivery profile. In its unexpanded configuration, distal apexes 2004 and anchor pins 2008 define distal spindle pin catches 2012.

Spindle pin catches 2012 are pockets, sometimes called openings or holes, in which proximal spindle pins 2030 are located to radially constrain distal anchor stent ring 1908 in its unexpanded configuration (crimped profile) prior to deployment as discussed in greater detail below. Generally, anchor pins 2008 are positioned slightly proximal from distal apexes 2004 to leave room for proximal spindle pins 2030.

A distal portion of distal anchor stent ring 1908 is restrained within middle member sleeve 2022. Middle member sleeve 2022 is illustrated as a transparent sleeve in FIG. 20 to illustrate features within middle member sleeve 2022. However, in other examples, middle member sleeve 2022 is opaque. Illustratively, middle member sleeve 2022 is stainless steel, Nitinol, MP35N alloy, or a polymer, or a combination thereof.

Proximal spindle pins 2030 of spindle 120G extend into and are located within spindle pin catches 2012 of distal anchor stent ring 1908. Accordingly, the distal end of distal anchor stent ring 1908 is locked around proximal spindle pins 2030 and between middle member sleeve 2022 and spindle body 122G. Generally, spindle 120G and middle member sleeve 2022 form a distal capture and release mechanism for distal anchor stent ring 1908.

Further, middle member sleeve 2022 holds anchor pins 2008 down (radially inward) thus providing a minimal delivery profile for distal anchor stent ring 1908. Generally, middle member sleeve 2022 holds anchor pins 2008 bent in a lower profile.

Middle member sleeve 2022 does not cover (exposes) proximal tips 2010 of anchor pins 2008. Stated another way, middle member sleeve 2022 extends proximally only partially over anchor pins 2008. This prevents proximal tips 2010, e.g., sharp tips, from engaging (digging into, scratching, gouging) middle member sleeve 2022. This minimizes the deployment force necessary to retract middle member sleeve 2022 relative to distal anchor stent ring 1908. However, in another example, a distal anchor stent ring similar to distal anchor stent ring 1908 without anchor pins 2008 is formed.

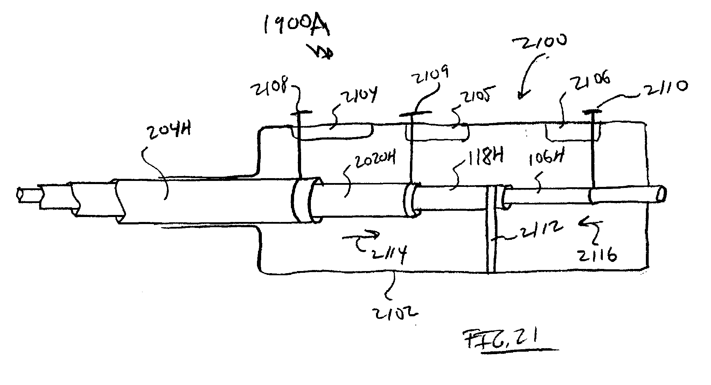

FIG. 21 is a handle 2100 of a stent-graft delivery system 1900A in accordance with one embodiment. Handle 2100 includes a housing 2102 having a primary sheath retraction slot 2104, a middle member retraction slot 2105, and an inner tube advancement slot 2106.

A primary sheath actuation member 2108, sometimes called a thumb slider, extends from a primary sheath 204H and through primary sheath retraction slot 2104. Similarly, a middle member actuation member 2109, sometimes called a thumb slider, extends from a middle member 2020H and through middle member retraction slot 2105. Further, an inner tube actuation member 2110, sometimes called a thumb slider, extends from an inner tube 106H and through inner tube advancement slot 2106. Further, an outer tube 118H is mounted to housing 2102 by an outer tube support 2112.

To retract primary sheath 204H relative to outer tube 118H, primary sheath actuation member 2108 is moved (retracted), e.g., by the physician, in the direction of arrow 2114. To retract middle member 2020H relative to outer tube 118H, middle member actuation member 2109 is moved (retracted), e.g., by the physician, also in the direction of arrow 2114. To advance inner tube 106H relative to outer tube 118H, inner tube actuation member 2110 is moved (advanced), e.g., by the physician, in the direction of arrow 2116.

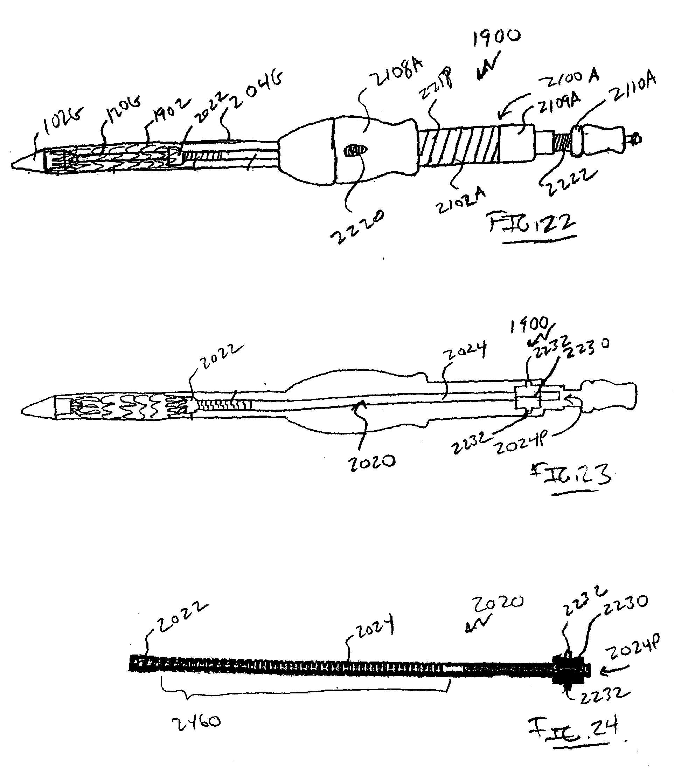

Although one example of a handle is set forth in FIG. 21, in light of this disclosure, those of skill in the art will understand that other handles can be used. Illustratively, handles having ratcheting mechanisms, threaded mechanisms, or other mechanisms to retract the primary sheath/middle member and advance the inner tube relative to the outer tube are used such as the handle illustrate in FIG. 22.

FIG. 22 is stent-graft delivery system 1900 of FIGS. 19, 20 including a handle 2100A. Handle 2100A includes a housing 2102A, a primary sheath actuation member 2108A, sometimes called an external slider, a middle member actuation member 2109A, sometimes called a moveable rear grip, and an inner tube actuation member 2110A, sometimes called a rear wheel similar to housing 2102, primary sheath actuation member 2108, middle member actuation member 2109 and inner tube actuation member 2110 of handle 2100 of FIG. 21. Only the significant differences between handle 2100A of FIG. 22 and handle 2100 of FIG. 21 are discussed below.

Primary sheath actuation member 2108A is coupled to primary sheath 204G through housing 2102A in such a manner that primary sheath actuation member 2108A can be rotated without rotation of primary sheath 204G yet longitudinal motion of primary sheath actuation member 2108A causes an equal longitudinal motion of primary sheath 204G. More particularly, primary sheath actuation member 2108A is threadedly attached to screw gear 2218 of housing 2102A. Rotation of primary sheath actuation member 2108A on screw gear 2218 causes axial translation of primary sheath actuation member 2108A and thus primary sheath 204G. Further, a primary sheath actuation member release 2220 selectively disengages primary sheath actuation member 2108A from screw gear 2218 allowing the physician to quickly retract primary sheath actuation member 2108A and thus primary sheath 204G with a single pull.

Similarly, inner tube actuation member 2110A is coupled to inner tube 106G (shown in FIG. 19) in such a manner that inner tube actuation member 2110A can be rotated without rotation of inner tube 106G yet longitudinal motion of inner tube actuation member 2110A causes an equal longitudinal motion of inner tube 106G. More particularly, inner tube actuation member 2110A is threadedly attached to a second screw gear 2222 of housing 2102A. Rotation of inner tube actuation member 2110A on screw gear 2222 causes axial translation of inner tube actuation member 2110A and thus inner tube 106G and tapered tip 102G.

Further, spindle 120G is mounted to housing 2102A, e.g., by an outer tube and outer tube support similar to outer tube 118H and outer tube 2112 of handle 2100 of FIG. 21.

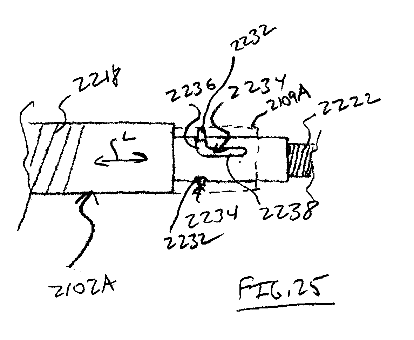

FIG. 23 is a partial cross-sectional view of stent-graft delivery system 1900 of FIG. 22. FIG. 24 is a perspective view of middle member 2020 of stent-graft delivery system 1900 of FIGS. 22 and 23. FIG. 25 is a perspective view of a portion of housing 2102A of stent-graft delivery system 1900 of FIGS. 22 and 23.

Referring now to FIGS. 22, 23, 24 and 25 together, middle member actuation member 2109A is coupled to middle member 2020 in such a manner that longitudinal motion of middle member actuation member 2109A causes an equal longitudinal motion of middle member 2020. To facilitate the coupling of middle member actuation member to middle member 2020, middle member 2020 includes a middle member lock 2230 at a proximal end 2024P of middle member tube 2024.

Middle member lock 2230 includes a pair of radially protruding posts 2232 opposite one another. Housing 2102A includes a pair of lock slots 2234 through which posts 2232 of middle member lock 2230 pass to be connected to middle member actuation member 2109A.

Lock slots 2234 include circumferential slot portions 2236 extending along the outer cylindrical surface of housing 2102A perpendicularly to a longitudinal axis L of handle 2100A. Lock slots 2234 further include longitudinal slot portions 2238 extending along the outer cylindrical surface of housing 2102A parallel to longitudinal axis L of handle 2100A.

By locating posts 2232 within circumferential slot portions 2236 of lock slots 2234, longitudinal motion of posts 2232 is prevented effectively locking middle member 2020 to housing 2102A. However, by rotating middle member actuation member 2109A and thus middle member 2020 to position posts 2232 within longitudinal slot portions 2238 of lock slots 2234, longitudinal motion of posts 2232 is enabled effectively unlocking middle member 2020 from housing 2102A.

As further illustrated in FIG. 24, middle member tube 2024 is formed with a flexible section 2460 having folds that provide flexibility to middle member tube 2024. In one example, middle member 2020 is formed of a solid molded polymer. In another example, middle member tube 2024 is formed of a solid molded polymer and middle member sleeve 2022 is stainless steel, Nitinol, or MP35N alloy molded into middle member tube 2024.

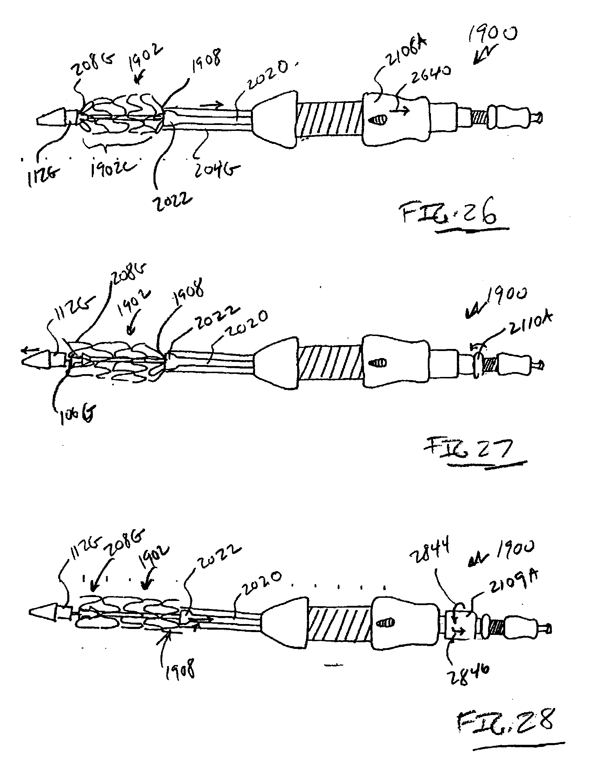

FIGS. 26, 27, 28 are side views of stent-graft delivery system 1900 of FIGS. 22 and 23 at various stages during deployment of stent-graft 1902. Referring to FIG. 26, once stent-graft delivery system 1900 is tracked and positioned at the target anatomy, primary sheath actuation member 2108A is moved (retracted), e.g., by the physician, in the direction of arrow 2640 as discussed above. This retracts primary sheath 204G deploying the central section 1902C of stent-graft 1902 between proximal anchor stent ring 208G and distal anchor stent ring 1908. However, proximal anchor stent ring 208G and distal anchor stent ring 1908 of stent-graft 1902 remained captured within sleeve 112G and middle member sleeve 2022, respectively. In one example, stent-graft 1902 is repositioned after deployment of central section 1902C.

Referring now to FIG. 27, inner tube actuation member 2110A is moved (advanced), e.g., by the physician as discussed above. This advances inner tube 106G and thus sleeve 112G deploying proximal anchor stent ring 208G of stent-graft 1902. In one example, deployment of proximal anchor stent ring 208G sets the final position of stent-graft 1902.

Referring now to FIG. 28, middle member actuation member 2109A is initially rotated in the direction of arrow 2844 to position posts 2232 within longitudinal slot portions 2238 of lock slots 2234 (see FIGS. 23-25) to unlock middle member 2020 from housing 2102A. Middle member actuation member 2109A is then moved (retracted), e.g., by the physician, in the direction of arrow 2846. This retracts middle member 2020 and thus middle member sleeve 2022 deploying distal anchor stent ring 1908 of stent-graft 1902. In one example, middle member actuation member 2109A is returned (advanced) back to its original longitudinal position, e.g., using a spring mechanism.

Once stent-graft 1902 is completely deployed, the delivery system is withdrawn from the patient.

Although FIGS. 26, 27, 28 illustrate deployment of central section 1902C, followed by deployment of proximal anchor stent ring 208G, followed by deployment of distal anchor stent ring 1908, it is to be understood that the three deployment phases are distinct and interchangeable and can be carried out in any desired order.

By providing stent-graft delivery system 1900 with a proximal capture and release mechanism for controlled deployment of proximal anchor stent ring 208G and a distal capture and release mechanism for controlled deployment of distal anchor stent ring 1908, deployment of stent-graft 1902 occurs in three distinct operations as illustrated in FIGS. 26, 27 and 28. This provides maximum control in the deployment of stent-graft 1902.

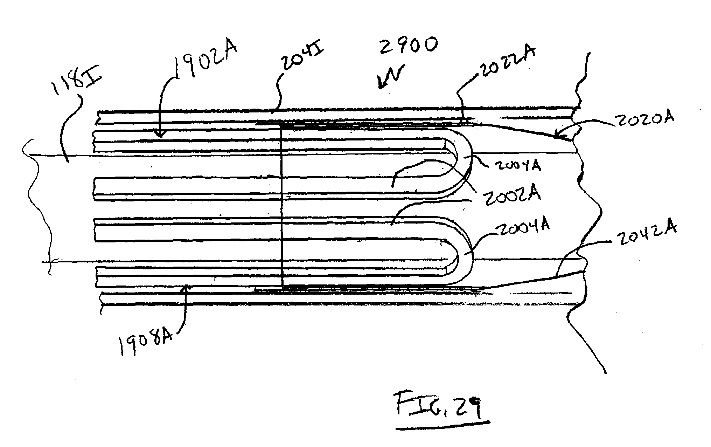

FIG. 29 is an enlarged side view of a region of a stent-graft delivery system 2900 in accordance with one embodiment. Referring now to FIG. 29, a stent-graft 1902A includes a distal anchor stent ring 1908A including struts 2002A and distal apexes 2004A. In accordance with this example, distal anchor stent ring 1908A has an absence of (does not include) anchor pins and spindle pin catches.

Stent-graft delivery system 2900 further includes a middle member 2020A having a middle member sleeve 2022A extending distally from a middle member tube 2024A of middle member 2020A similar to middle member 2020 of FIGS. 19 and 20.

Stent-graft delivery system 2900 further includes outer tube 1181 having a spindle (not shown) located at and fixed to the distal end of outer tube 1181 similar to spindle 120 of FIG. 1. An annular space exists between outer tube 1181 and middle member sleeve 2022A. Stent-graft delivery system 2900 further includes a primary sheath 204I.

Distal anchor stent ring 1908A is illustrated in its unexpanded configuration, sometimes called delivery profile. Generally, distal anchor stent ring 1908A is restrained within middle member sleeve 2022A. More particularly, distal anchor stent ring 1908A is restrained within the annular space existing between outer tube 118I and middle member sleeve 2022A.

In a manner similar to that discussed above in reference to FIG. 28, middle member 2020A and thus middle member sleeve 2022A are retracted to release distal anchor stent ring 1908A of stent-graft 1902A, which self-expands upon release.

.

The drawings and the forgoing description gave examples of embodiments according to the present invention. Numerous variations, whether explicitly given in the specification or not, such as differences in structure, dimension, and use of material, are possible.

## Claims

1. A delivery system comprising:
a spindle (120G) comprising:
a spindle body (122G);
distal spindle pins (124G) extending radially outward from said spindle body (122G); and
and/or proximal spindle pins (2030) extending radially outward from said spindle body (122G);
a tip (1 02G) comprising a sleeve (112G), wherein said distal spindle pins (124G), if provided, extend from said spindle body (122G) toward said sleeve;
an middle member (2020) comprising a middle member sleeve (2022), wherein said proximal spindle pins (2030), if provided, extend from said spindle body toward said middle member sleeve (2022); and
an endoprosthesis (1902) comprising proximal spindle pin catches (1202) at a proximal end of said endoprosthesis and/or distal spindle pin catches (2012) at a distal end (202D) of said endoprosthesis (1902), the proximal spindle pin catches (1202) being adapted so that said distal spindle pins (124G) extend into said proximal spindle pin catches (1202), and/or the distal spindle pin catches (2012) being adapted so that said proximal spindle pins (2030) extend into said distal spindle pin catches (2012).

2. The delivery system of Claim 1 wherein said middle member (2020) further comprises a middle member tube (2024) connected to said middle member sleeve (2022).

3. The delivery system of Claim 2 wherein said middle member sleeve (2022) extends distally from said middle member tube (2024).

4. The delivery system of any of the preceding claims wherein said middle member sleeve (2022) comprises a cylindrical outer surface (2026) and a cylindrical inner surface (2028), said spindle body (122G) comprising a cylindrical outer surface.

5. The delivery system of Claim 4 wherein a diameter to which said proximal spindle pins (2030) extend from said cylindrical outer surface of said spindle body (122G) is approximately equal to or slightly less than a diameter of said inner cylindrical surface (2028) of said middle member sleeve (2022).

6. The delivery system of Claim 4 or 5 wherein an annular space (128G) exists between said spindle body (122G) and said inner cylindrical surface (2028) of said middle member sleeve (2022).

7. The delivery system of Claim 6 wherein said endoprosthesis (1902) further comprises a distal anchor stent ring (1908) in said annular space (128G).

8. The delivery system of any of the preceding claims further comprising an inner tube (106G), said tip (102G) being mounted on said inner tube (106G).

9. The delivery system of any of the preceding claims further comprising:
an outer tube (118G), said spindle (120G) being mounted to said outer tube (118G), wherein said inner tube (106G) is within and extends through said outer tube (118G) and said spindle (120G), said inner tube (106G) and tip (102G) capable of being longitudinally moved relative to said outer tube (118G) and said spindle (120G).

10. The delivery system of Claim 9 further comprising a handle (2100) comprising an inner tube actuation member (2110) for advancing said inner tube (106G) relative to said outer tube (118G).

11. The delivery system of Claim 9 or 10 wherein said middle member (2020) is a hollow tube and defines a lumen therein through which said outer tube (118G) and said inner tube (106G) extend.

12. The delivery system of Claim 11 further comprising a handle (2100) comprising a middle member actuation member (2109) for retracting said middle member (2020) relative to said outer tube (118G).

13. The delivery system of Claim 11 or 12 further comprising a primary sheath (204H) over said middle member (2020), said outer tube (118H) and said inner tube (106H), said primary sheath (204H) capable of being longitudinally moved relative to said outer tube (118H) and said spindle (120G).

14. The delivery system of Claim 13 further comprising a handle (2100) comprising a primary sheath actuation member (2108) for retracting said primary sheath (204H) relative to said outer tube (118H).

15. The delivery system of Claim 13 or 14 wherein said primary sheath (204H) radially compresses said endoprosthesis (1902).

16. The delivery system of any of the preceding claims wherein said endoprosthesis (1902) comprises a proximal anchor stent ring (208G) comprising:
proximal apexes;
struts extending from said proximal apexes; and
a pair of anchor pins located on said struts adjacent each of said proximal apexes, said proximal apexes and said anchor pins defining said proximal spindle pin catches.

17. The delivery system of any of the preceding claims wherein said endoprosthesis (1902) comprises a distal anchor stent ring (1908) comprising:
distal apexes (2004);
struts (2002) extending from said distal apexes (2004); and
a pair of anchor pins (2008) located on said struts (2002) adjacent each of said distal apexes (2004), said distal apexes (2004) and said anchor pins (2008) defining said distal spindle pin catches (2012).

18. The delivery system of Claim 17 wherein said anchor pins (2008) comprise proximal tips (2010) uncovered by said middle member sleeve (2022).

19. The delivery system of Claim 18 wherein said proximal tips (2010) are sharp points.
